# FASCICULE DE BREVET EUROPEEN

(11) **EP 1 006 946 B1**
(45) Date de publication et mention de la délivrance du brevet: **22.01.2003**
(21) Numéro de dépôt: 99926544.0
(22) Date de dépôt: 25.06.1999
(51) Int. Cl.: A61F 2/06

(54) **SYSTEME DE POSITIONNEMENT TRIDIMENSIONNEL DE MATERIELS PAR VOIE ENDOLUMINALE**
DREIDIMENSIONALES POSITIONIERSYSTEM FÜR ENDOLUMINAL EINGEBRACHTES MATERIAL
SYSTEM FOR THREE-DIMENSIONAL POSITIONING OF INSTRUMENTS BY INTRALUMINAL TRACT

(30) Priorité: 25.06.1998 FR 9808092
(43) Date de publication de la demande: 14.06.2000
(73) Titulaire: Bergeron, Patrice, 13009 Marseille (FR)
(72) Inventeur: Bergeron, Patrice, 13009 Marseille (FR)
(74) Mandataire: Breese, Pierre
(86) Numéro de dépôt international: FR9901537
(87) Numéro de publication internationale: WO99066861

(56) Documents cités:
- EP-A- 0 804 907
- EP-A- 0 891 751
- WO-A-96/34580
- WO-A-97/16217
- WO-A-98/19628
- US-A- 5 653 690
- US-A- 5 749 825

## Description

La présente invention concerne le domaine de l'angioplastie.

Elle concerne plus particulièrement un dispositif destiné à introduire un matériel, par exemple un stent, dans le réseau artériel par voie endoscopique.

L'invention vise à résoudre le problème du positionnement d'un matériel tel qu'un stent biseauté au niveau d'une bifurcation. Le stent biseauté présente un biais qui doit être positionné angulairement de façon à optimiser la circulation sanguine.

On connaît dans l'état de la technique différentes solutions pour poser des stents bifurqués. A titre d'exemple, la demande dé brevet WO 98/19628 décrit un stent biseauté et un équipement constitué par un ballonnet guidé par un fil de guidage. Un cathéter de stabilisation guidé par un deuxième fil de guidage est destiné à préparer le positionnement d'une bifurcation. Il s'agit de deux moyens indépendants, qui ne permettent pas de positionner de façon simple et efficace l'instrument d'introduction du stent bifurqué, ni d'assurer de façon ergonomique le positionnement angulaire de l'élément d'introduction du stent dans la branche latérale.

Une autre demande de brevet, publiée sous le numéro EP891751, divulgue un ballonnet comportant une spire venant se positionner dans l'artère principale, pour faciliter le positionnement d'un ballonnet dans une voie latérale. Cette demande de brevet a été déposée le 19 juillet 1997 et publiée le 20 janvier 1999. Elle fait donc partie de l'état de la technique au sens des articles 54.3 et 54.4 de la Convention sur le Brevet Européen pour tous les états désignés, sauf Chypre.

Une autre demande de brevet, publiée sous le numéro WO 97/16217, divulgue un ballonnet gonflable en Y, formé en une seule pièce.

L'invention concerne un dispositif de positionnement d'un matériel introduit dans le réseau artériel par voie endoscopique selon la revendication 1.

Ce prolongement asymétrique permet un positionnement angulaire du dispositif par rapport à l'orientation de la bifurcation.

Selon un premier mode de réalisation, l'élément tubulaire est constitué par un ballonnet gonflable.

Avantageusement, l'élément tubulaire est formé par un ballonnet gonflable prolongé par une excroissance latérale.

Selon un deuxième mode de réalisation, l'élément tubulaire est prolongé par un ergot latéral pour le positionnement angulaire du matériel.

Selon une variante préférée, le prolongement latéral présente un repère radio-contrasté.

Selon une autre variante avantageuse, le dispositif comporte un moyen de retenue pour maintenir le prolongement latéral en position repliée contre l'élément tubulaire pendant la phase d'introduction.

Selon un exemple de mise en oeuvre, le moyen de retenue est constitué par un lien susceptible d'être libéré par traction sur un fil.

Selon une variante de réalisation, le dispositif de positionnement comporte un manchon amovible entourant, au moment de l'introduction dans l'artère, le stent et l'excroissance latérale, ledit manchon pouvant être retiré à proximité de la bifurcation pour libérer l'ergot latéral.

De préférence, le manchon amovible comporte un marqueur radio-opaque asymétrique.

L'invention sera mieux comprise à la lecture qui suit, se référant à un exemple non limitatif de réalisation où :

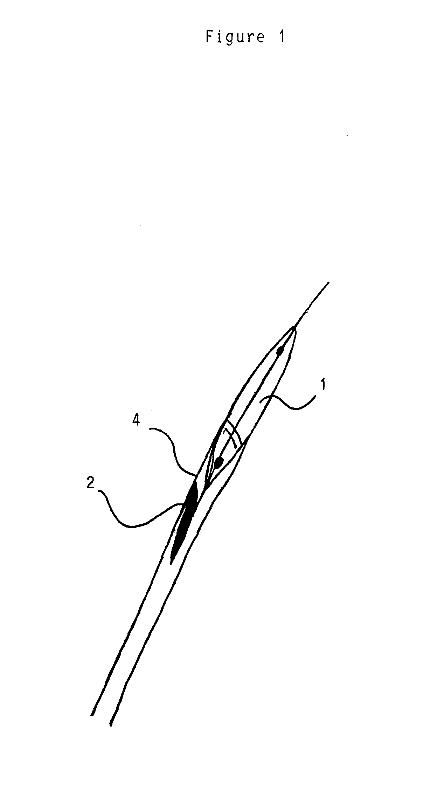

- la figure 1 représente un dispositif pendant la phase d'introduction ;
- la figure 2 représente un dispositif pendant la phase de positionnement ;
- la figure 3 représente une vue en coupe d'une variante de réalisation ;
- les figures 4 et 5 représentent des vues de variantes de réalisation, après retrait du manchon amovible ;
- les figures 6 à 9 représentent d'autres variantes de réalisation.

Le dispositif est constitué par un élément tubulaire (1) formant un ballonnet sensiblement cylindrique. Ce ballonnet est avantageusement gonflable pour assurer une fonction accessoire d'expansion d'un stent.

Il est prolongé à son extrémité distale par un prolongement latéral asymétrique (2) formé par un ergot.

Pendant la phase d'introduction par voie endoscopique, l'ergot est replié en position axiale. Il peut être maintenu par une gaine (4) qui maintient l'ergot en position effacée.

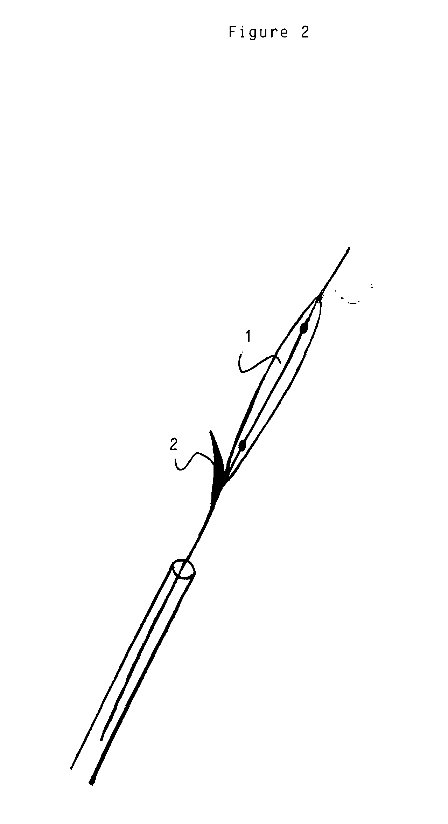

Cette gaine (4) est rattachée à un fil permettant de la retirer en direction proximale pour libérer l'ergot, comme représenté en figure 2.

L'ergot vient alors s'orienter selon une direction radiale. Il constitue un repère angulaire permettant le positionnement du dispositif et du matériel qu'il supporte, par exemple un stent biseauté. L'ergot présente un repère radio-contrasté permettant de vérifier l'orientation et de positionner correctement le stent biseauté.

Il peut également être réalisé sous forme d'un ballonnet principal de forme tubulaire, prolongé par une excroissance latérale gonflable séparément.

Il peut encore être réalisé sous forme d'un élément tubulaire prolongé à l'extrémité distale par un ergot en silicone ou par un ergot gonflable.

L'ergot peut être proximal ou distal par rapport au ballon ou autres systèmes porteur du stent, si celui-ci est autoexpansible à mémoire de forme.

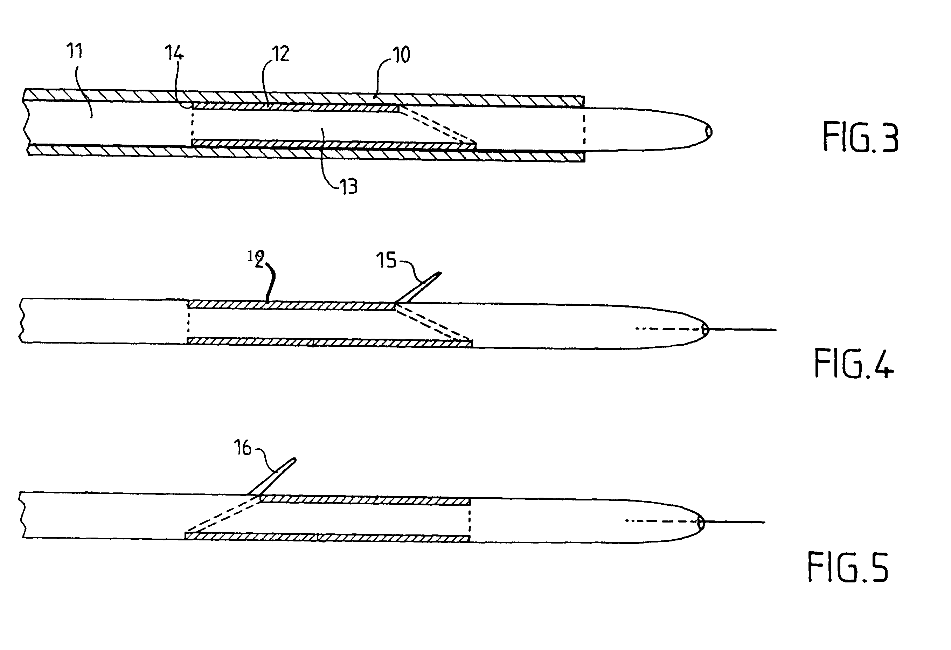

La figure 3 représente une vue en coupe d'une variante de réalisation avec un manchon amovible (10), en position d'introduction, et les figures 4 et 5 représentent des vues de variantes de réalisation, après retrait du manchon amovible.

Le cathéter (11) forme un support du stent biseauté (12). Ce cathéter (11) présente à cet effet une partie distale (13) dont la section correspond à la section intérieure de l'endoprothèse en position retractée. Un épaulement (14) assure le blocage du stent (12).

Le manchon (10) peut être retiré par traction sur un fil fixé sur l'extrémité dudit manchon. Le retrait du manchon (10) libère l'ergot latéral (15 ou 16), qui peut être en position distale, en avant du stent (12), comme représenté en figure 4, ou en position proximale, en arrière du stent (12), comme représenté en figure 5.

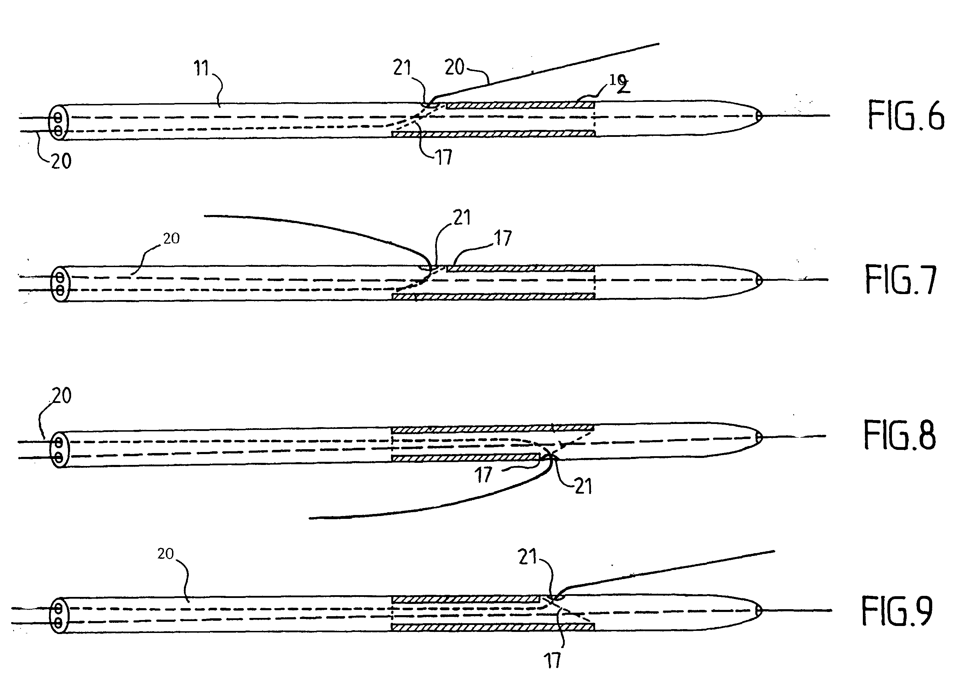

Les figures 6 à 9 représentent d'autres variantes de réalisation, avec un guide métallique (20) traversant le cathéter porteur (11) et ressortant en zone proximale par une lumière (21) qui peut se trouver en arrière du stent comme représenté en figures 6 et 7, ou en avant du stent comme représenté en figures 8 et 9. La lumière (20) est prévue dans la zone en biais (17) du stent destinée au raccordement avec la branche fille.

Le guide (20) est introduit dans la branche fille de la bifurcation artérielle. Il peut être dirigé dans le sens antégrade ou dans le sens rétrograde selon la configuration du chenal et de son orifice ménagé dans le cathéter (11).

Les marqueurs, ergots ou guide sont maintenus contre le cathéter porteur (11) grâce au manchon extérieur (10) pendant l'étape d'introduction dans l'artère. Le retrait du manchon rétractable (10) provoque l'érection de l'ergot, et permet l'avancement du guide (20). Le retrait du manchon peut être réalisé du côté proximal ou distal.

## Revendications

1. Dispositif de positionnement d'un stent (12) biseauté au niveau d'une bifurcation, ledit stent étant introduit dans le réseau artériel par voie endoscopique **caractérisé en ce qu'**il est constitué par un élément tubulaire (1), ledit élément tubulaire (1) présentant un prolongement latéral asymétrique (2) pour le positionnement angulaire du stent par rapport à l'orientation de ladite bifurcation, ledit dispositif comportant en outre une gaine (4) maintenant ledit prolongement latéral asymétrique (2) en position effacée pendant la phase d'introduction par voie endoscopique, ladite gaine étant attachée à un fil, pour libérer ledit prolongement latéral asymétrique (2).

2. Dispositif de positionnement d'un stent (12) biseauté dans le réseau artériel selon la revendication 1 **caractérisé en ce que** ledit fil permet de retirer ladite gaine (4) en direction proximale.

3. Dispositif de positionnement d'un stent (12) biseauté dans le réseau artériel selon la revendication 1 ou la revendication 2 caractérisé en ce l'élément tubulaire est constitué par un ballonnet gonflable.

4. Dispositif de positionnement d'un stent (12) biseauté dans le réseau artériel selon la revendication 3 **caractérisé en ce que** le ballonnet est prolongé par une excroissance latérale gonflable séparément constituant le prolongement latéral asymétrique.

5. Dispositif de positionnement d'un stent (12) biseauté dans le réseau artériel selon l'une au moins des revendications précédentes, **caractérisé en ce que** l'élément tubulaire (1) est prolongé par un ergot latéral pour le positionnement angulaire du stent, constituant ledit prolongement latéral asymétrique.

6. Dispositif de positionnement d'un stent (12) biseauté dans le réseau artériel selon l'une au moins des revendications précédentes, **caractérisé en ce que** le prolongement latéral asymétrique (2) présente un repère radio-contrasté.

## Patentansprüche

1. Vorrichtung zur Positionierung eines an einer Bifurkation abgeschrägten Stents (12), wobei der besagte Stent in das arterielle Netz über den endoskopischen Weg eingeführt wird, **dadurch gekennzeichnet, dass** er aus einem rohrförmigen Element (1) besteht, wobei das besagte rohrförmige Element eine asymmetrische seitliche Verlängerung (2) zur winkeligen Positionierung des Stents bezüglich der Orientierung der besagten Bifurkation aufweist, wobei die besagte Vorrichtung ferner eine Hülle (4) aufweist, welche die besagte asymmetrische seitliche Verlängerung (2) während der über den endoskopischen Weg erfolgenden Einführungsphase in Null-Stellung hält, wobei die besagte Hülle an einen Faden befestigt ist, um die besagte asymmetrische Verlängerung (2) zu befreien.

2. Vorrichtung zur Positionierung eines abgeschrägten Stents (12) im arteriellen Netz gemä_ Anspruch 1, **dadurch gekennzeichnet, dass** der besagte Faden es ermöglicht, die besagte Hülle (4) in proximaler Richtung zu entfernen.

3. Vorrichtung zur Positionierung eines abgeschrägten Stents (12) im arteriellen Netz gemä_Anspruch 1 oder 2, **dadurch gekennzeichnet, dass** das rohrförmige Element aus einem aufblasbaren Ballon besteht.

4. Vorrichtung zur Positionierung eines abgeschrägten Stents (12) im arteriellen Netz gemä_ Anspruch 3, **dadurch gekennzeichnet, dass** der Ballon durch eine seitliche, getrennt aufblasbare Ausstülpung verlängert ist, welche die asymmetrische seitliche Verlängerung bildet.

5. Vorrichtung zur Positionierung eines abgeschrägten Stents (12) im arteriellen Netz gemä_ mindestens einem der vorstehenden Ansprüche, **dadurch gekennzeichnet, dass** das rohrförmige Element (1) durch einen seitlichen Nocken zur winkeligen Positionierung des Stents verlängert ist, welcher die besagte asymmetrische seitliche Verlängerung bildet.

6. Vorrichtung zur Positionierung eines abgeschrägten Stents (12) im arteriellen Netz gemäß mindestens einem der vorstehenden Ansprüche, **dadurch gekennzeichnet, dass** die asymmetrische seitliche Verlängerung eine Röntgenkontrast bildende Markierung aufweist.

## Claims

1. A device for positioning a beveled stent (12) at the level of a bifurcation, said stent being introduced into the arterial arc by endoscopic means, **characterized in that** it is comprised of a tubular member (1), said tubular member (1) having an asymmetrical lateral extension (2) for the angular positioning of the stent relative to the orientation of said bifurcation, said device further comprising a sheath (4) holding said asymmetrical lateral extension (2) in a hidden position during the introduction phase by endoscopic route, said sheath being attached to a wire for releasing said asymmetrical lateral extension (2).

2. A device for positioning a beveled stent (12) in the arterial arc according to Claim 1, wherein said wire enables retracting said sheath (4) in the proximal direction.

3. A device for positioning a beveled stent (12) according to Claim 1 or Claim 2, wherein the tubular member is comprised of an inflatable balloon.

4. A device for positioning a beveled stent (12) in the arterial arc according to the Claim 3, wherein the balloon is elongated by a lateral inflatable excrescence separately comprising the asymmetrical lateral extension.

5. A device for positioning a beveled stent (12) in the arterial arc according to at least one of the above claims, wherein the tubular member (1) is extended by a lateral pin for angular positioning of the stent comprising said asymmetrical lateral extension.

6. A device for positioning a beveled stent (12) in the arterial arc according to at least one of the above Claims, wherein the asymmetrical lateral extension (2) has a radio-contrast marker.
